# FASCICULE DE BREVET EUROPEEN

(11) **EP 2 408 901 B1**
(45) Date de publication et mention de la délivrance du brevet: **27.05.2015**
(21) Numéro de dépôt: 10716395.8
(22) Date de dépôt: 16.03.2010
(51) Int. Cl.: C12M 1/26

(54) **RECIPIENT-MELANGEUR AVEC PALIER D'ARBRE EN PARTIE SUPÉRIEURE.**
MISCHBEHÄLTER MIT EINEM WELLENLAGER IM OBEREN TEIL
MIXING CONTAINER COMPRISING A SHAFT BEARING IN THE UPPER PART

(30) Priorité: 18.03.2009 FR 0951746
(43) Date de publication de la demande: 25.01.2012
(73) Titulaire: SARTORIUS STEDIM FMT SAS, 13400 Aubagne (FR)
(72) Inventeur: GUENERON, Maréva, F-13112 La Destrousse (FR)
(74) Mandataire: Derambure, Christian
(86) Numéro de dépôt international: PCT/FR2010/050466
(87) Numéro de publication internationale: WO 2010/106282

(56) Documents cités:
- EP-A- 1 884 561
- WO-A-90/11347
- US-A- 4 670 397
- US-A1- 2006 270 036

## Description

L'invention est relative au domaine des récipients-mélangeurs.

Elle vise plus particulièrement un récipient-mélangeur destiné à recevoir un contenu biopharmaceutique en vue de son mélange et un tel récipient-mélangeur faisant fonction de bioréacteur.

Le document US-A-2006/0270036 décrit un récipient-mélangeur qui comprend un dispositif rigide extérieur de contention, un conteneur flexible disposé dans le logement, des moyens de mélange du contenu et des moyens d'aération.

Selon une réalisation, l'arbre est descendant, le palier étant supérieur, et les moyens d'aération sont localisés à l'aplomb même des moyens de mélange. Selon une autre réalisation, l'arbre, de très petite dimension axiale est montant, le palier étant inférieur, et les moyens de mélange forment une sorte de couronne placée autour du palier.

Un tel récipient-mélangeur présente comme inconvénient que la structure limite le mélange obtenu et se révèle particulièrement inapproprié dans le cas où l'on souhaite avoir un conteneur de grande capacité, par exemple pouvant atteindre 5.000 litres.

Le document US 4 670 397 est relatif à un fermenteur. Le document EP-A-1884561 est relatif à un récipient de culture avec ventilation. Le document WO 90/11347 est relatif à un oxygénateur statique pour une culture en suspension.

L'invention vise à résoudre les problèmes posés par les récipients-mélangeurs connus du type comportant des moyens d'aération, et, plus particulièrement, à optimiser tant le mélange que l'aération et ce, y compris dans le cas de conteneurs de grande capacité par exemple pouvant atteindre 5.000 litres.

A cet effet, l'invention vise un récipient-mélangeur destiné à recevoir un contenu biopharmaceutique en vue de son mélange, comprenant:
▪ un conteneur flexible, comportant :
   ∘ une paroi ayant une partie inférieure, une partie latérale et une partie supérieure, délimitant un espace intérieur apte à recevoir une certaine quantité du contenu,
   ∘ un ou plusieurs ports d'introduction dans le conteneur du contenu ou de composants du contenu, coopérant avec un ou plusieurs orifices d'introduction ménagés dans le conteneur,
   ∘ au moins un port de vidange du contenu coopérant avec au moins un orifice de vidange,
▪ des moyens de mélange du contenu, comportant :
   ∘ au moins un arbre, apte à être entraîné à rotation par des moyens moteur et à entraîner à rotation au moins un organe de mélange,
   ∘ au moins un palier avec lequel coopère une partie d'extrémité de l'arbre,
   ∘ au moins un organe de mélange, apte à agiter le contenu, situé dans l'espace intérieur, caractérisé par le fait qu'il comporte au moins un port combiné introduction/palier supérieur ayant une flasque rigide :
      ∘ pourvue d'un passage d'introduction en communication fluidique d'un côté avec l'espace intérieur et de l'autre avec l'extérieur du conteneur,
      ∘ fixée de façon rigide et étanche à la partie supérieure de la paroi du conteneur autour d'un orifice d'introduction, le passage d'introduction et l'orifice d'introduction étant en communication fluidique,
      ∘ supportant du côté intérieur un palier supérieur situé dans l'espace intérieur, adjacent au passage d'introduction sans empêcher la communication fluidique entre le passage d'introduction et l'ouverture d'introduction.

Selon une caractéristique, le récipient-mélangeur comporte en outre des moyens d'aération aptes à délivrer au contenu une certaine quantité de gaz d'aération, comportant des moyens d'amenée de gaz d'aération ayant au moins un élément tubulaire s'étendant avec communication fluidique depuis l'extérieur du conteneur jusqu'aux moyens de distribution, et des moyens de distribution de gaz d'aération comprenant au moins un élément étendu de distribution dont la paroi laisse passer des bulles du gaz d'aération provenant des moyens d'amenée, situé dans l'espace intérieur vers la partie inférieure de la paroi du conteneur.

Selon une réalisation, le au moins un arbre est maintenu par le seul palier supérieur et s'étend sur une partie seulement de la distance entre la partie supérieure et la partie inférieure de la paroi du conteneur, le moyen moteur d'entraînement à rotation de l'arbre étant situé vers la partie supérieure de la paroi du conteneur.

Selon une réalisation, le moins un organe de mélange est substantiellement espacé de la partie inférieure et de la partie supérieure de la paroi du conteneur.

Selon une réalisation, le au moins un arbre des moyens de mélange est situé tout entier dans l'espace intérieur, le moyen moteur d'entraînement à rotation de l'arbre étant à fonctionnement magnétique, un disque rotatif menant à pôles magnétiques, situé à l'extérieur du conteneur, coopérant fonctionnellement avec un disque rotatif mené à pôles magnétiques, fixé sur le au moins un arbre à proximité magnétique du disque rotatif menant.

Selon une réalisation, le au moins un arbre des moyens de mélange est situé pour partie dans l'espace intérieur et pour partie à l'extérieur du conteneur, le moyen moteur d'entraînement à rotation de l'arbre étant à fonctionnement mécanique, un arbre rotatif menant, situé à l'extérieur du conteneur, coopérant fonctionnellement avec la partie extérieure du au moins un arbre.

Selon une réalisation, les moyens de mélange comportent un unique arbre descendant.

Selon une réalisation, les moyens de mélange comportent plusieurs arbres descendant d'axes substantiellement parallèles, aptes à entraîner à rotation chacun au moins un organe de mélange.

Selon une réalisation, un arbre des moyens de mélange supporte et entraîne un unique organe de mélange situé en une unique localisation axiale sur l'arbre.

Selon une réalisation, un arbre des moyens de mélange supporte et entraîne plusieurs organes de mélange situés en une pluralité de localisations axiales sur l'arbre.

Selon une réalisation, un organe de mélange est substantiellement espacé de la partie supérieure de la paroi du conteneur, d'une distance de l'ordre d'au moins le tiers de l'écartement entre la partie inférieure et la partie supérieure de la paroi du conteneur.

Selon une réalisation, le au moins un élément tubulaire d'amenée de gaz d'aération s'étend dans l'espace intérieur, en étant substantiellement maintenu attenant ou adjacent à la face intérieure de la paroi du conteneur.

Selon une réalisation, le au moins un élément tubulaire d'amenée de gaz d'aération est au moins pour partie structurellement distinct de la paroi du conteneur et maintenue à elle par collage, soudage ou au moyen de pièces de maintien rapportées.

Selon une réalisation, le au moins un élément tubulaire d'amenée de gaz d'aération est au moins pour partie structurellement partie intégrante de la paroi du conteneur.

Selon une réalisation, le au moins un élément tubulaire d'amenée de gaz d'aération traverse la paroi du conteneur par une liaison étanche.

Selon une réalisation, le au moins un élément tubulaire d'amenée de gaz d'aération traverse la paroi du conteneur dans la partie supérieure.

Selon une réalisation, le au moins un élément étendu de distribution de gaz d'aération est maintenu attenant ou adjacent à la face intérieure de la partie inférieure de la paroi du conteneur.

Selon une réalisation, le au moins un élément étendu de distribution de gaz d'aération est, au moins pour partie, structurellement distinct de la paroi du conteneur et maintenu à elle par collage, soudage ou au moyen de pièces de maintien rapportées.

Selon une réalisation, le au moins un élément étendu de distribution de gaz d'aération est, au moins pour partie, structurellement partie intégrante de la paroi du conteneur.

Selon une réalisation, le au moins un élément étendu de distribution de gaz d'aération ne traverse pas la paroi du conteneur.

Selon une réalisation, le au moins un élément étendu de distribution de gaz d'aération comporte une paroi pourvue d'une pluralité de trous répartis aptes au passage des bulles du gaz d'aération provenant des moyens d'amenée.

Selon une réalisation, la pluralité de trous aptes au passage des bulles du gaz d'aération provenant des moyens d'amenée est orientée avec différents axes d'inclinaison sur la verticale.

Selon une réalisation, les trous de la pluralité de trous sont soit de même taille soit de tailles différentes.

Selon une réalisation, le au moins un élément étendu de distribution de gaz d'aération a, en section droite transversale, une forme circulaire, ou pseudo-circulaire, ou elliptique ou pseudo-elliptique.

Selon une réalisation, le au moins un élément étendu de distribution de gaz d'aération comprend au moins un anneau complet fermé sur lui-même, en communication circulaire continue ou non.

Selon une réalisation, le au moins un élément étendu de distribution de gaz d'aération comprend au moins un anneau incomplet ouvert par rapport à lui-même.

Selon une réalisation, l'anneau incomplet a une ouverture d'angle comprise entre environ 180° et 270°.

Selon une réalisation, le au moins un élément étendu de distribution de gaz d'aération comprend au moins un anneau et au moins un élément transversal en communication fluidique.

Selon une réalisation, le au moins un anneau du au moins un élément étendu de distribution de gaz d'aération est sensiblement centré sur le port de vidange.

Selon une réalisation, les moyens d'aération comportent un unique ensemble de moyens d'amenée de gaz d'aération et de moyens de distribution de gaz d'aération.

Selon une réalisation, les moyens d'aération comportent plusieurs ensembles distincts de moyens d'amenée d'un ou de plusieurs gaz d'aération et de moyens de distribution du ou des gaz d'aération.

Selon une réalisation, un ensemble de moyens d'aération comporte un seul élément tubulaire d'amenée de gaz d'aération communiquant avec un seul élément étendu de distribution de gaz d'aération, ou un seul élément tubulaire d'amenée de gaz d'aération communiquant avec plusieurs éléments étendus de distribution de gaz d'aération, ou plusieurs éléments tubulaires d'amenée de gaz d'aération communiquant avec un seul élément étendu de distribution de gaz d'aération, ou plusieurs éléments tubulaires d'amenée de gaz d'aération communiquant avec plusieurs éléments étendus de distribution de gaz d'aération.

Selon une réalisation, il comporte plusieurs éléments étendus de distribution de gaz d'aération distincts, caractérisé par le fait que au moins certains des plusieurs éléments étendus de distribution de gaz d'aération sont situés en une pluralité de localisations radiales dans l'espace intérieur vers la partie inférieure du conteneur.

Selon une réalisation, les plusieurs éléments étendus de distribution de gaz d'aération distincts sont substantiellement espacés radialement du port de vidange jusqu'au voisinage de la partie latérale de la paroi du conteneur.

Selon une réalisation, un élément étendu de distribution de gaz d'aération est substantiellement espacé radialement du port de vidange d'une distance de l'ordre d'au moins le cinquième du diamètre de la partie inférieure de la paroi du conteneur.

Selon une réalisation, ne saille sous la partie inférieure de la paroi du conteneur que la vidange.

Selon une réalisation, il comporte également un ou plusieurs ports d'évacuation de gaz coopérant avec au moins un orifice d'évacuation ménagé dans la partie supérieure de la paroi du conteneur, pourvu d'une valve anti-retour, empêchant l'introduction dans le conteneur de fluides ou de contaminants non souhaités ou indésirables.

Selon une réalisation, il comporte également un ou plusieurs ports d'introduction, de vidange, de montage.

Selon une réalisation, le conteneur est de grande capacité, pouvant aller jusqu'à 5.000 litres.

Selon une réalisation, il comporte également un dispositif rigide extérieur de contention du conteneur empli de son contenu, comprenant une paroi de fond, une paroi périphérique et une ouverture supérieure, délimitant un logement principal dans lequel est placé de façon amovible le conteneur flexible dont la partie inférieure repose sur la paroi de fond et dont la partie latérale vient s'appliquer, lorsque le conteneur est empli de son contenu, contre la paroi périphérique.

Selon une réalisation, le dispositif rigide extérieur de contention comporte également un logement secondaire en dessous de la paroi de fond de logement et de protection de la vidange et, le cas échéant, du moyen moteur d'entraînement des moyens de mélange lorsqu'il est prévu en partie inférieure.

Selon une réalisation, le dispositif rigide extérieur de contention comporte également des moyens de chauffage et le conteneur flexible est en un matériau présentant une certaine conductivité thermique, de manière que la mise en oeuvre des moyens de chauffage permette le chauffage du contenu ; et, le cas échéant, des moyens de contrôle de la température du conteneur et des moyens de commande des moyens de chauffage.

Selon une réalisation, le conteneur peut se trouver dans trois états extrêmes: un état désassemblé du dispositif rigide extérieur de contention dans lequel le conteneur peut être disposé de façon aplatie sur lui-même, un état assemblé au dispositif rigide extérieur de contention dans lequel le conteneur, vide de contenu, est disposé dans le logement principal du dispositif de contention en reposant sur la paroi de fond, et un état assemblé au dispositif rigide extérieur de contention dans lequel le conteneur, empli de son contenu, est disposé dans le logement principal du dispositif de contention en reposant sur la paroi de fond et en étant appliqué contre la paroi périphérique.

Selon une réalisation, on y réalise une bioréaction, le récipient-mélangeur étant un bioréacteur.

On décrit maintenant plusieurs modes de réalisation de l'invention à l'aide des dessins, dans lesquels :
- La figure 1 est une vue en perspective d'une réalisation possible d'un récipient-mélangeur, dont le dispositif rigide extérieur de contention n'est pas représenté.
- Les figures 2A, 2B et 2C sont trois vues en perspective de trois modes de réalisation d'un port combiné introduction/palier supérieur destiné à faire partie du récipient-mélangeur.

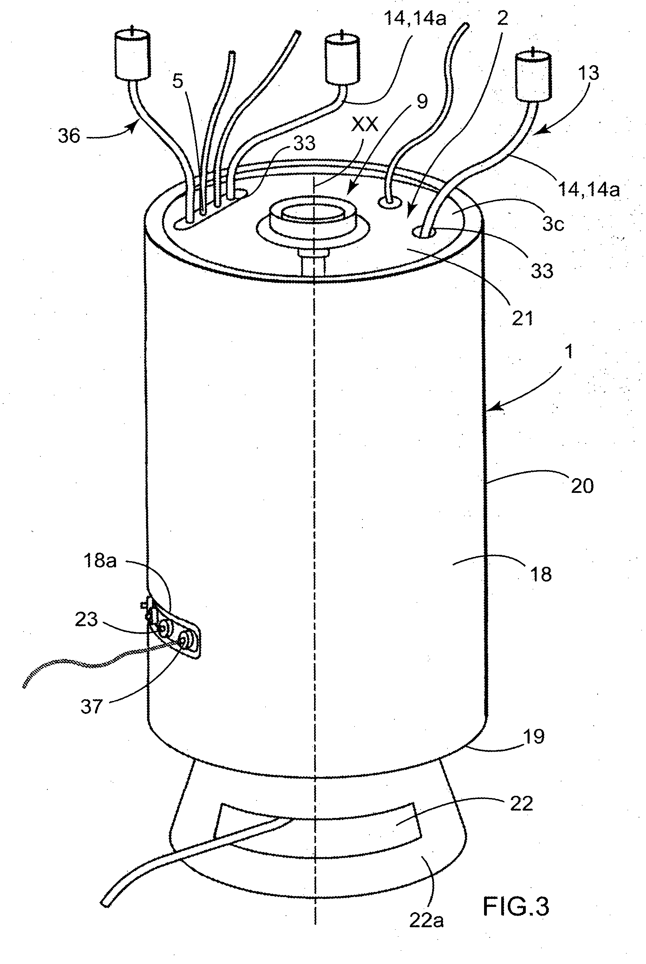

- La figure 3 est une vue en perspective extérieure du récipient-mélangeur montrant le dispositif rigide extérieur de contention.

Un récipient-mélangeur 1 selon l'invention est destiné à recevoir un contenu biopharmaceutique C en vue de son mélange ou, le cas échéant en vue d'une bioréaction, le récipient-mélangeur 1 étant alors un bioréacteur.

Le contenu C comprend une ou au moins une phase liquide. Le cas échéant, le contenu C est réalisé à partir de plusieurs composants C₁, C₂... dont au moins un est en phase liquide et dont un ou plusieurs peut être en phase solide, tel que de la poudre. Le cas échéant, dans le cas d'un bioréacteur, le contenu C comprend également des cellules, des micro-organismes...

Le récipient-mélangeur 1 présente un axe principal XX, vertical.

Le récipient-mélangeur 1 comporte en premier lieu un conteneur flexible 2. Ce conteneur flexible 2 est formé par une paroi 3, en un ou plusieurs tronçons solidarisés les uns avec les autres, ayant une partie inférieure 3a, une partie latérale 3b et une partie supérieure 3c, délimitant un espace intérieur 4, apte à recevoir une certaine quantité du contenu C.

Selon une réalisation, le conteneur flexible 2 est à usage unique.

Le conteneur flexible 2 peut avoir une capacité allant jusqu'à 5.000 litres, en fonction des besoins et des applications.

Les mots « vertical », « horizontal », « supérieur », « inférieur » se réfèrent à la situation dans laquelle le récipient-mélangeur 1 est dans une position apte à son fonctionnement. Il est entendu toutefois que le récipient-mélangeur 1 peut occuper d'autres positions ou avoir d'autres états, par exemple parce qu'il n'est pas en fonctionnement. Le mot « vertical » ne doit pas être compris dans un sens étroit, mais dans le sens signifiant du plus haut au plus bas et inversement.

D'autre part, les mots « intérieur » et « extérieur » se réfèrent, respectivement à ce qui se trouve dans et hors du conteneur 2.

Enfin, les mots « axial » d'une part, « radial » et « transversal » d'autre part, se réfèrent à ce qui s'étend dans ou parallèlement ou sensiblement parallèlement à l'axe XX d'une part, et perpendiculairement ou orthogonalement ou sensiblement perpendiculairement ou orthogonalement à l'axe XX d'autre part.

Le récipient-mélangeur 1 comporte un ou plusieurs ports 5 traversant, d'introduction dans le conteneur 2 du contenu C ou de composants C₁, C₂... du contenu C, coopérant avec un ou plusieurs orifices d'introduction ménagés dans le conteneur 2.

Le récipient-mélangeur 1 comporte également au moins un port 6 traversant de vidange du contenu C du conteneur 2, coopérant avec au moins un orifice de vidange ménagé dans le conteneur 2. Naturellement, le port de vidange 6 est apte à être obturé à chaque fois que nécessaire et au contraire ouvert pour la vidange.

On entend dans ce document par « port », un moyen de connexion ou de liaison physique. Un tel port est traversant lorsqu'il s'agit d'assurer une fonction de mise en communication entre l'intérieur et l'extérieur du conteneur 2, par exemple pour l'introduction ou la vidange de ce qui doit être placé ou est placé dans le conteneur 2. Un tel port peut également être non traversant lorsqu'il s'agit d'assurer une fonction de maintien d'un organe du récipient-mélangeur.

Aux ports 5 peuvent être associé, en communication fluidique et avec une connexion étanche et le cas échéant amovible, des conduits, poches, réservoirs, le cas échéant souples. De même, aux ports 6 peuvent être associé, en communication fluidique et avec une connexion étanche et le cas échéant amovible, des conduits, poches, réservoirs, le cas échéant souples. Ces conduits, poches, réservoirs 5b et 6b sont situés et s'étendent à l'extérieur du récipient-mélangeur 1 et reliés de façon appropriée à des amenées et évacuations, respectivement. Ces conduits, poches, réservoirs sont adaptés - notamment en ce qui concerne leur taille - à la nature de ce qu'ils contiennent ou assurent le passage. La connexion étanche et le cas échéant amovible est assurée par tout dispositif approprié, comme il est connu dans le domaine de l'invention.

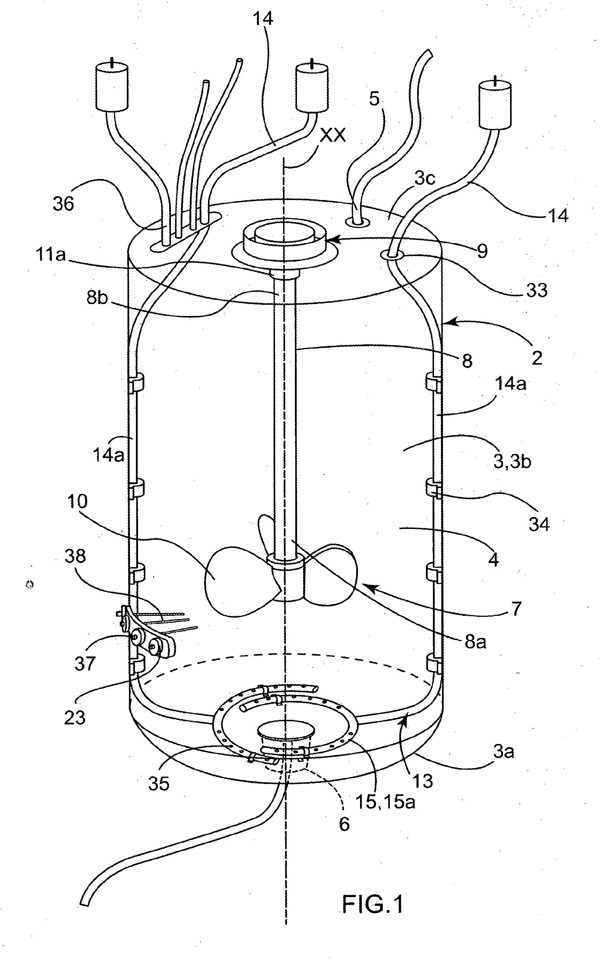

Dans la réalisation représentée sur la figure 1, les ports 5 d'introduction sont placés en position supérieure du récipient-mélangeur 1 et les orifices d'introduction sont ménagés dans la partie supérieure 3c de la paroi 3, tandis que le port 6 de vidange est placé en position la plus basse du récipient-mélangeur 1 et l'orifice de vidange ménagé dans la partie inférieure 3a du conteneur 2, dans sa zone la plus basse. Le cas échéant, un (ou plusieurs) port 5 d'introduction est placé en position inférieure du récipient-mélangeur 1 et l'orifice d'introduction correspondant ménagé dans la partie inférieure 3a du conteneur 2 ou dans la zone inférieure de la partie latérale 3b.

Le récipient-mélangeur 1 comporte également des moyens 7 de mélange du contenu du conteneur 2. On entend par là le mélange de ce qui se trouve dans l'espace intérieur 4 du conteneur 2, qu'il s'agisse du contenu C, ou d'une partie de ses composants, et/ou d'une partie seulement de la quantité totale qui doit y être placée.

Les moyens 7 de mélange comprennent, en premier lieu, au moins un arbre 8, descendant, apte à être entraîné à rotation par des moyens moteurs 9 et à entraîner à rotation au moins un organe de mélange 10.

Les moyens 7 de mélange comprennent, en deuxième lieu, au moins un palier supérieur 11a, adjacent à la partie supérieure 3c de la paroi 3, avec lequel coopère la partie supérieure 8b de l'arbre 8.

Les moyens 7 de mélange comprennent, en troisième lieu, au moins un organe de mélange 10, apte à agiter le contenu, situé dans l'espace intérieur 4.

Le récipient-mélangeur 1 comporte également des moyens d'aération 13 aptes à délivrer au contenu une certaine quantité de gaz d'aération. On entend par là l'aération de ce qui se trouve dans l'espace intérieur 4 du conteneur 2, qu'il s'agisse du contenu C; ou d'une partie de ses composants, et/ou d'une partie seulement de la quantité totale qui doit y être placée.

Les moyens 13 d'aération comprennent, en premier lieu, des moyens 14 d'amenée de gaz d'aération ayant au moins un élément tubulaire 14a s'étendant avec communication fluidique depuis l'extérieur du conteneur 2 jusqu'à des moyens de distribution 15.

Les moyens 13 d'aération comprennent, en second lieu, les moyens 15 de distribution de gaz d'aération comprenant au moins un élément étendu 15a de distribution dont la paroi laisse passer des bulles du gaz d'aération provenant des moyens d'amenée 14. Cet élément étendu 15a de distribution de gaz d'aération est situé dans l'espace intérieur 4, vers la partie inférieure 3a de la paroi 3 du conteneur 2.

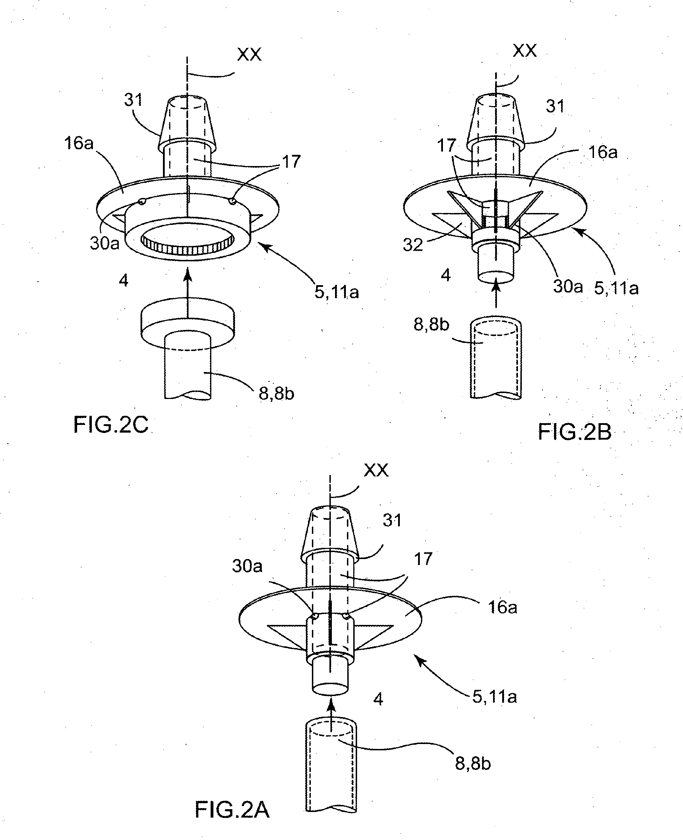

Le récipient-mélangeur 1 comporte également dans la réalisation représentée sur les figures 2A, 2B et 2C, un port combiné introduction/palier supérieur 5+11a ayant une flasque rigide 16a.

On entend ici par « flasque », une pièce rigide en forme générale de paroi pleine, au moins sensiblement plate, placée à plat, et destinée au maintien.

Dans ce cas, cette flasque 16a est, en premier lieu, pourvue d'un passage 17 d'introduction du contenu C ou de composants C₁, C₂... du contenu C, ce passage 17 étant en communication fluidique d'un côté avec l'extérieur du conteneur 2 et de l'autre avec l'espace intérieur 4.

Cette flasque 16a est, en deuxième lieu, fixée de façon rigide et étanche à la partie supérieure 3c de la paroi 3 du conteneur 2 autour de l'orifice d'introduction prévu à cet effet, le passage d'introduction et l'orifice d'introduction étant en communication fluidique.

Cette flasque 16a, en troisième lieu, supporte du côté intérieur le palier supérieur 11a situé dans l'espace intérieur 4 en étant adjacent au passage d'introduction, sans empêcher la communication fluidique entre le passage d'introduction et l'orifice d'introduction.

Le au moins un élément étendu 15a de distribution de gaz d'aération est substantiellement espacé radialement du port de vidange 6. On entend par là que l'élément étendu 15a n'est pas incorporé dans ou à proximité immédiate du port de vidange 6.

Le au moins un élément tubulaire 14a de distribution de gaz d'aération s'étend à partir de l'élément étendu 15a de distribution, dans l'espace intérieur 4, le long de la face intérieure de la partie inférieure 3a et de la partie latérale 3b de la paroi 3 du conteneur 2. Le au moins un élément tubulaire 14a de distribution de gaz d'aération s'étend à l'extérieur du conteneur 2 à partir de la - ou du voisinage de la - partie supérieure 3c de la paroi 3 du conteneur 2.

Au moins un organe de mélange 10 est substantiellement espacé de la partie inférieure 3a de la paroi 3 du conteneur 2 et du au moins un élément étendu 15a de distribution de gaz d'aération. On entend par là que l'organe de mélange 10 n'est pas incorporé dans ou à proximité immédiate de la paroi 3 du conteneur 2, et du au moins un élément étendu 15a de distribution dé gaz d'aération.

Les dispositions constructives qui précèdent sont telles que les bulles du gaz d'aération distribuées depuis le au moins un élément étendu 15a de distribution du gaz d'aération, sont réparties dans le contenu de l'espace intérieur 4 par, d'une part, une première répartition dans la zone inférieure de l'espace intérieur 4 adjacente à la partie inférieure 3a de la paroi 3 du conteneur 2, par le au moins un élément étendu 15a de distribution du gaz de distribution et, d'autre part, une seconde répartition par le au moins un organe de mélange 10 dans l'ensemble de l'espace intérieur 4 du conteneur 2.

Le récipient-mélangeur 1 comporte également, en raison de la nature flexible du conteneur 2, un dispositif rigide - éventuellement semi-rigide - extérieur de contention 18 du conteneur 2 empli de son contenu pendant le remplissage, le mélange et la vidange.

Le dispositif rigide extérieur de contention 18 comprend une paroi de fond 19 et une paroi périphérique 20, ménageant une ouverture supérieure d'accès 21 et délimitant un logement principal dans lequel est placé de façon amovible le conteneur flexible 2.

Le dispositif rigide extérieur de contention 18 est généralement de géométrie, forme et et/ou dimension identiques au conteneur flexible 2, afin de réduire les sollicitations sur les soudures ou les changements de direction dans la matière du conteneur flexible 2.

Le dispositif rigide extérieur de contention 18 comporte l'ouverture d'accès 21 afin de permettre la mise en place et l'enlèvement du conteneur flexible 2.

Le cas échéant, le dispositif rigide extérieur de contention 18 comporte d'autres ouvertures pour introduire le contenu C ou les composants C₁, C₂... du contenu C et vidanger le contenu C, ou pour accéder aux différents éléments du récipient-mélangeur 1 qui doivent être accessibles pour l'utilisation.

La partie inférieure 3a de la paroi 3 du conteneur 2 repose sur la paroi de fond 19, tandis que la partie latérale 3b de la paroi 3 du conteneur 2 vient s'appliquer, lorsque le conteneur 2 est empli de son contenu, contre la paroi périphérique 20.

Le cas échéant, le dispositif rigide extérieur de contention 18 comporte également, ou forme, un logement ou espace secondaire 22 situé en dessous de la paroi de fond 19. Ce logement ou espace secondaire 22 permet le logement et la protection des moyens du récipient-mélangeur 1 se trouvant en dessous du conteneur 2. Il s'agit par exemple du conduit, de la poche ou du réservoir associé au port de vidange. Le cas échant, le logement ou espace secondaire 22 peut être ménagé à l'intérieur d'un piètement 22a, tel que tant la partie inférieure 3a de la paroi 3 du conteneur 2 que la paroi de fond 19 du dispositif rigide extérieur de contention 18 soit écarté du sol ou de la surface d'appui recevant le récipient-mélangeur 1, ce dernier étant maintenu en position verticale, tout en autorisant l'accès à l'orifice de vidange.

Le cas échéant, le dispositif rigide extérieur de contention 18 comporte également des moyens de chauffage destinés au chauffage du contenu du conteneur 2. Dans ce cas, le conteneur flexible est réalisé en un matériau présentant une certaine conductivité thermique, de manière que la mise en oeuvre des moyens de chauffage en question permette le chauffage du contenu. Dans ce cas, et le cas échéant, il est également prévu des moyens de contrôle de la température du contenu dans le conteneur 2 et des moyens de commande des moyens de chauffage. De tels moyens de contrôle de la température sont portés par un ou des ports 23 prévus à cet effet.

Le cas échéant, le dispositif rigide extérieur de contention 18 comporte également des portes, des fenêtres, etc. 18a

Selon une réalisation, la paroi de fond 19 a une forme de calotte arrondie, par exemple hémisphérique ou pseudo hémisphérique, la partie inférieure 3a de la paroi 3 du conteneur 2 ayant une même forme. Cette disposition constructive, combinée aux dispositions constructives précédemment exposées, contribue à l'efficacité du mélange et de l'aération.

Le conteneur flexible 2 peut se trouver dans trois états extrêmes :
- Un état désassemblé, dans lequel le conteneur 2 est désassemblé du dispositif rigide extérieur de contention 18. Dans cet état, le conteneur 2, qui est flexible dans son ensemble, peut - alors qu'il est vide du contenu C - être disposé de façon aplatie sur lui-même. Cet état est tout particulièrement utile pour le stockage ou le transport.
- Un état assemblé vide, dans lequel le conteneur 2 est assemblé au dispositif rigide extérieur de contention 18, comme il a été décrit plus haut. le conteneur 2 étant vide du contenu C. Dans cet état, le conteneur 2 est disposé dans le logement principal du dispositif de contention 18 en reposant sur la paroi de fond 19.
- et, enfin, un état assemblé empli, dans lequel le conteneur est assemblé au dispositif rigide extérieur de contention 18, comme il a été décrit plus haut, le conteneur 2 étant empli de contenu C. Dans cet état, le conteneur 2 est disposé dans le logement principal du dispositif de contention 18 en reposant sur la paroi de fond 19 et en étant appliqué contre la paroi périphérique 20.

Pour le procédé de mise en oeuvre d'un récipient-mélangeur 1 tel qu'il vient d'être décrit, on dispose d'un tel récipient-mélangeur 1 dont le port de vidange 6 est obturé et on dispose du contenu C ou des composants C₁, C₂... du contenu C.

Ce contenu C ou ces composants C₁, C₂... du contenu C sont destinés à être reçus dans le conteneur du récipient-mélangeur 1, puis mélangé, moyennant une aération.

Selon le procédé, tout d'abord, on introduit dans le conteneur 2, le contenu C ou les composants C₁, C₂... du contenu C, par le ou les ports 5.

Puis, on met en oeuvre les moyens 7 de mélange pour agiter le contenu du conteneur 2 se trouvant dans l'espace intérieur 4.

Par ailleurs, on met en oeuvre les moyens 13 d'aération pour délivrer au contenu du conteneur 2 se trouvant dans l'espace intérieur 4, une certaine quantité de gaz d'aération.

L'agitation et l'aération sont réalisées au moins partiellement simultanément, le cas échéant, totalement simultanément.

Par suite des dispositions constructives du récipient-mélangeur 1 et du procédé mis en oeuvre, on distribue les bulles du gaz d'aération depuis le au moins un élément étendu 15a de distribution et on les répartit dans le contenu C se trouvant dans l'espace intérieur 4, par, d'une part, une première répartition dans la zone inférieure de l'espace intérieur 4 adjacente à la partie inférieure 3a de la paroi 3 du conteneur 2, par le au moins un élément étendu 15a de distribution du gaz de distribution et, d'autre part, une seconde répartition par le au moins un organe de mélange 10 dans l'ensemble de l'espace intérieur 4 du conteneur 2.

Le procédé peut faire l'objet de plusieurs modes d'exécution. Ainsi, on peut introduire, d'abord dans le conteneur 2 un composant Cᵢ du contenu C ou une partie des composants C₁, C₂... du contenu C, puis on met en oeuvre les moyens 7 de mélange et les moyens 13 d'aération de manière à délivrer au contenu une certaine quantité de gaz d'aération, puis on introduit dans le conteneur 2 le ou les composants C₁, C₂... restant du contenu C.

Le récipient-mélangeur 1 comportant d'une part le conteneur flexible 2 et d'autre part le dispositif rigide extérieur de contention 18, on peut procéder ainsi.

On part d'un récipient-mélangeur 1 dont le conteneur 2 est désassemblé du dispositif rigide extérieur de contention 18, ainsi que vide de contenu et disposé de façon plus ou moins aplatie sur lui-même.

Puis, on assemble le conteneur 2 au dispositif rigide extérieur de contention 18, en le disposant dans le logement principal de celui-ci, en reposant sur sa paroi de fond 19.

Enfin, on introduit alors dans le conteneur 2 le contenu C ou des composants C₁, C₂... du contenu C.

Le reste du procédé est comme décrit plus haut.

Le récipient-mélangeur peut faire l'objet de plusieurs réalisations en fonction des différentes variantes d'exécution des moyens 7 de mélange, du port combiné introduction/palier supérieur 5+11a et des moyens 13 d'aération, les différentes variantes de ces moyens 7 et 13 pouvant, de surcroît, être le plus souvent combinées entre elles.

On décrit maintenant plus spécialement différentes variantes d'exécution des moyens 7 de mélange.

Dans la réalisation de l'invention, le au moins un arbre 8 des moyens 7 de mélange coopère avec un unique palier, à savoir le palier supérieur 11a. L'arbre 8 comporte une extrémité libre inférieure 8a qui est écartée de la partie inférieure 3a de la paroi 3 du conteneur 2, par exemple située environ à mi-hauteur du conteneur 2. Le moyen moteur 9 d'entraînement à rotation de l'arbre 8 est situé vers la partie supérieure 3c de la paroi 3 du conteneur 2.

Le palier supérieur 11a a une flasque 16a, rigide, fixée de façon rigide à la partie supérieure 3c de la paroi 3 du conteneur 2. Cette flasque 16a supporte du côté intérieur le palier supérieur 11a qui est situé dans l'espace intérieur 4.

Selon une variante d'exécution possible, le au moins un arbre 8 des moyens 7 de mélange est situé tout entier dans l'espace intérieur 4. Dans ce cas, le moyen moteur 9 d'entraînement à rotation de l'arbre 8, vers la partie supérieure 3c de la paroi 3 du conteneur 2, est à fonctionnement magnétique. A cet effet, il est prévu un disque rotatif menant situé à l'extérieur du conteneur 2, coopérant fonctionnellement avec un disque rotatif mené à pôles magnétiques, fixé sur le au moins un arbre 8 à proximité magnétique du disque rotatif menant.

L'extrémité supérieure 8b de l'arbre 8 incorpore ainsi un disque magnétique incluant une pluralité d'aimants, qui est intégré par tout moyen de fixation ou de construction. Le disque magnétique est ensuite positionné à proximité de la flasque supérieure 16a. Le disque magnétique est raccordé à la flasque supérieure, de manière à permettre au moyen moteur magnétique d'agir sur les aimants du disque magnétique, dans la largeur de la flasque supérieure 16a.

Par exemple, le disque magnétique est fixé sur l'arbre 8 par vissage d'une extrémité filetée de l'arbre 8 dans une ouverture filetée à l'intérieur du disque magnétique. D'autres moyens, tels qu'éléments clavetés, adhésifs, attaches, fixations rapides, goupilles, vis, verrous, soudage, et similaires, ainsi que la formation du disque magnétique sur l'arbre 8 lors de sa fabrication, peuvent être utilisés pour fixer le disque magnétique à l'arbre 8, sans limitation.

Pour maintenir le disque magnétique en relation correcte avec la flasque supérieure, il est prévu un crochet ou un cliquet sur le disque magnétique, qui s'engage sur une lèvre de la flasque supérieure 16a. Les moyens particuliers de fixation du disque magnétique et de la flasque supérieure 16a consistant en l'utilisation du cliquet associé à la flasque supérieure 16a et de la lèvre associée au disque magnétique ne sont pas exclusifs d'autres, des alternatives telles que fixations rapides, profilés et similaires étant possibles, dès lors que le disque magnétique peut tourner relativement librement par rapport à la flasque supérieure 16a.

De plus, pour s'assurer que le moyen moteur 9, magnétique conserve un alignement correct avec les aimants du disque magnétique, la flasque supérieure 16a inclut un accouplement d'entraînement qui s'étend vers le haut, à partir de la face extérieure de la flasque supérieure 16a.

Selon une autre variante d'exécution possible, le au moins un arbre 8 des moyens 7 de mélange est situé pour partie dans l'espace intérieur 4 et pour partie à l'extérieur du conteneur 2, une liaison tournante étanche étant prévue. Dans ce cas, le moyen moteur 9 d'entraînement à rotation de l'arbre 8 peut être à fonctionnement mécanique, un arbre rotatif menant, situé à l'extérieur du conteneur 2, coopérant fonctionnellement avec la partie extérieure du au moins un arbre 8.

Selon une réalisation, les moyens 7 de mélange comportent un unique arbre 8 descendant. On entend par « descendant », le fait que l'arbre 8 s'étend généralement dans une direction haut-bas ou verticale à partir du palier supérieur 11a.

Selon une autre réalisation possible, les moyens 7 de mélange comportent plusieurs arbres 8 d'axes substantiellement parallèles entre eux et tous attenants à la partie supérieure 3c de la paroi 3 du conteneur 2. Ces arbres 8 sont aptes à entraîner à rotation chacun au moins un organe de mélange 10. Le récipient-mélangeur 1 comporte dans cette réalisation plusieurs organes de mélange 10.

Selon une réalisation possible, un arbre 8 des moyens 7 de mélange supporte et entraîne un unique organe de mélange 10 situé en une unique localisation axiale sur l'arbre 8. Selon une autre réalisation, un arbre 8 supporte et entraîne plusieurs organes de mélange 10 situés en une pluralité de localisations axiales sur l'arbre 8.

Un organe de mélange 10 peut se présenter sous la forme d'une hélice ayant un moyeu supportant plusieurs pales.

Un organe de mélange 10 est substantiellement espacé de la partie inférieure 3a de la paroi 3 du conteneur 2 et du au moins un élément étendu 15a de distribution. Selon une réalisation, cet espacement ou distance est de l'ordre d'au moins le quart de l'écartement entre la partie inférieure 3a et la partie supérieure 3c de la paroi 3 du conteneur 2. En particulier, cet espacement ou distance est de l'ordre d'au moins le tiers de l'écartement entre la partie inférieure 3a et la partie supérieure 3c de la paroi 3 du conteneur 2.

On décrit maintenant plus spécialement différentes variantes d'exécution du port combiné introduction/palier supérieur 5+11a.

La flasque 16a associée à la partie supérieure 8b de l'arbre 8 et faisant partie du récipient-mélangeur 1, est plus particulièrement représenté sur les figures 2A, 2B et 2C selon plusieurs variantes d'exécution. Dans chacune de ces variantes, la flasque 16a est formé d'une matière sensiblement rigide, de préférence une matière plastique rigide, en forme de paroi ou de plaquette raccordée au conteneur flexible 2 dans son axe XX, au centre de la partie supérieure 3c. Cette flasque 16a peut être raccordée au conteneur flexible 2 de toute manière appropriée de façon à former un joint rigide et hermétique entre les matières respectives, rigide et flexible.

La partie inférieure de la flasque supérieure 16a, qui se trouve dans l'espace intérieur 4 du conteneur 2, inclut le palier supérieur 11a qui forme un moyen de raccordement qui coopère avec la partie supérieure 8b de l'arbre 8.

Le palier 11a peut être un palier mâle en forme de tourillon, tel que représenté sur les figures 2A et 2B, inséré dans une cavité ouverte ménagée dans la partie supérieure 8b de l'arbre 8. Le palier 11a peut être un palier femelle en forme de couronne, tel que représenté sur la figure 2C, dans la cavité de laquelle est insérée la partie supérieure 8b de l'arbre 8.

Il est préféré que l'arbre 8 s'adapte sur le palier 11 avec un frottement minimal, de sorte que l'arbre 8 peut tourner librement sur le palier 11. A cet effet, on prévoit d'inclure possiblement un palier de butée entre la partie supérieure 8b de l'arbre 8 et le palier 11a, ou il peut être prévu des paliers lisses, à billes ou à rouleaux.

Si cela est souhaité, un cliquet, qui ne compromet pas significativement la rotation de l'arbre 8 sur le palier 11a, peut être prévu afin de maintenir l'arbre 8 sur le palier 11a.

Dans une réalisation, à la flasque supérieure 16a est associé un port d'introduction 5 avec un orifice d'introduction ménagé dans le conteneur 2. La flasque 16a est donc pourvue d'un passage d'introduction 30 en communication fluidique d'un côté avec l'extérieur du conteneur 2 et de l'autre avec l'espace intérieur 4 du conteneur 2, via le port d'introduction 5 proprement dit, ici en forme de tronçon de tube comportant une partie extrême comportant une saillie périphérique extérieure en forme de dent de requin 31, permettant la fixation de la partie extrême d'un conduit d'introduction.

Le passage d'introduction 30 comporte une ou plusieurs ouvertures 30a assurant la communication avec l'espace intérieur 4. Ces ouvertures 30a sont disposées en fonction de la structure du palier 11a.

Dans la variante de la figure 2A, il est prévu une pluralité d'ouvertures 30a radiales ou sensiblement radiales, ménagées à la base - supérieure - du palier 11a en forme de tourillon, réparties autour, et ces ouvertures 30a débouchent dans le port d'introduction 5.

Dans la variante de la figure 2B, il est prévu une ouverture 30a unique, axiale, ménagée axialement en dessus de la base - supérieure - du palier en forme de tourillon 11, laquelle base - supérieure - est abaissée par rapport à la flasque 16a au moyen d'entretoises 32 disposées radialement et axialement, un espace étant ainsi ménage entre cette base et la flasque 16.

Dans la variante de la figure 2C, il est prévu une pluralité d'ouvertures 30a radiales ou sensiblement radiales, ménagées à la base - supérieure - du palier 11a en forme de couronne, réparties autour, et ces ouvertures 30a débouchent dans le port d'introduction 5.

On décrit maintenant plus spécialement différentes variantes d'exécution des moyens 13 d'aération.

Le au moins un élément tubulaire 14a d'amenée de gaz d'aération s'étend dans l'espace intérieur 4 du conteneur, en étant substantiellement maintenu attenant ou adjacent ou contre la face intérieure de la paroi 3 du conteneur 2, de manière à éviter que l'élément tubulaire 14a ne divague dans le conteneur 2, alors que le contenu de celui-ci est agité par les moyens 7 de mélange, au risque d'interférer avec ceux-ci.

A cet effet, selon les différentes variantes d'exécution envisageables, le au moins un élément tubulaire 14a d'amenée de gaz d'aération est au moins pour partie structurellement distinct de la paroi 3 du conteneur 2 et maintenue à elle par collage, soudage ou au moyen de pièces de maintien 34, apportées, et/ou au moins pour partie structurellement partie intégrante de la paroi 3 du conteneur 2.

Des exemples typiques de pièces de maintien 34 sont des bandes d'adhésif, des brides, des cavaliers ou analogue disposées de place en place le long de l'élément tubulaire 14a d'amenée de gaz d'aération.

L'élément tubulaire 14a d'amenée peut également être un manchon. Ce manchon est construit en une longueur de matière, de préférence, identique à celle de la face intérieure de la paroi 3 du conteneur flexible 2, soudée ou fixée autrement sur ses côtés longitudinaux sur la face intérieure de la paroi 3 en étant placé à l'intérieur du conteneur flexible 2. La zone tubulaire comprise entre ce manchon et la face intérieure de la paroi 3 du conteneur flexible 2 achemine le gaz depuis l'extérieur du récipient-mélangeur jusqu'aux moyens 15 de distribution de gaz d'aération.

En amont (arrivée du gaz d'aération), le au moins un élément tubulaire 14a d'amenée de gaz d'aération traverse la paroi 3 du conteneur dans la partie supérieure 3c, par une liaison étanche 33. Cette disposition constructive permet, d'une part, de libérer la zone située au dessous de la partie inférieure 3a qui n'est pas encombrée par cet élément tubulaire 14a et, d'autre part, de ne pas être obligé de prévoir dans la partie inférieure 3a de la paroi 3 du conteneur 2, une ouverture de passage pour l'élément tubulaire 14a.

D'autre part, le au moins un élément étendu 15a de distribution de gaz d'aération est maintenu attenant ou adjacent à la face intérieure de la partie inférieure 3a de la paroi 3 du conteneur 2.

Selon les différentes variantes d'exécution envisageables, le au moins un élément étendu 15a de distribution de gaz d'aération est, au moins pour partie, structurellement distinct de la paroi 3 du conteneur 2 et maintenu à elle par collage, soudage ou au moyen de pièces de maintien rapportées et/ou au moins pour partie, structurellement partie intégrante de la paroi 3 du conteneur 2. En tout état de cause, le au moins un élément étendu 15a de distribution de gaz d'aération ne traverse pas la paroi 3 du conteneur 2. De telles pièces de maintien rapportées, lorsqu'elles sont prévues, peuvent être identiques ou analogues à celles utilisées pour l'élément tubulaire 14a d'amenée.

Bien entendu, la variante d'exécution du au moins un élément tubulaire 14a d'amenée de gaz d'aération est en adéquation avec celle du au moins un élément étendu 15a de distribution de gaz d'aération.

Le au moins un élément tubulaire 14a d'amenée de gaz d'aération s'étend, depuis l'amont vers l'aval, depuis l'extérieur vers l'intérieur du conteneur 2 et l'élément étendu 15a de distribution de gaz d'aération, par la liaison étanche 33 dans la partie supérieure 3c, puis axialement le long de la face intérieure de la partie latérale 3b jusqu'à la face intérieure de la partie inférieure 3a, puis radialement ou sensiblement radialement sur la face intérieure de la partie inférieure 3a, jusqu'à le au moins un élément étendu 15a de distribution de gaz d'aération attenant ou adjacent à la même face intérieure de la partie inférieure 3a de la paroi 3.

Le au moins un élément étendu 15a de distribution de gaz d'aération est du type comportant une paroi pourvue d'une pluralité de trous 35, répartis sur cette paroi. L'élément tubulaire 14a d'amenée de gaz d'aération débouche en communication fluidique d'un côté de cette paroi 15a, alors que l'autre côté de cette paroi 15a est située dans l'espace intérieur 4. Les trous 35 sont aptes au passage des bulles du gaz d'aération provenant des moyens 14 d'amenée vers l'espace intérieur 4.

Les trous 35 peuvent faire l'objet de différentes variantes d'exécution.

Selon une variante d'exécution possible, les trous 35 de la pluralité de trous 35 sont orientés avec différents axes d'inclinaison sur l'axe XX vertical.

Selon d'autres variantes d'exécution possible, les trous 35 de la pluralité de trous 35 sont soit de même taille soit de tailles différentes.

Ainsi, le débit du gaz d'aération sortant des trous 35 et l'orientation de sortie des bulles de gaz en aval de la paroi de l'élément étendu 15a de distribution de gaz d'aération peuvent être adaptés en fonction des besoins.

Ces réalisations s'appliquent aussi bien à une variante d'exécution selon laquelle les moyens 13 d'aération comportent un unique ensemble de moyens 14 d'amenée de gaz d'aération et de moyens 15 de distribution de gaz d'aération, et à une variante selon laquelle les moyens 13 d'aération comportent plusieurs ensembles distincts de moyens 14 d'amenée d'un ou de plusieurs gaz d'aération et de moyens 15 de distribution du ou des gaz d'aération.

Une telle disposition constructive à plusieurs ensembles distincts de moyens 14 d'amenée et de moyens 15 de distribution d'un ou de plusieurs gaz d'aération est particulièrement bien adaptée au cas où le procédé exige l'aération avec plusieurs gaz, par exemple de l'aération avec de l'oxygène et de l'aération avec du gaz carbonique.

Dans le cas d'une disposition à plusieurs ensembles distincts de moyens 14 d'amenée et de moyens 15 de distribution d'un ou de plusieurs gaz d'aération, les différents moyens 15 de distribution de la pluralité de moyens 15 de distribution ont soit les mêmes caractéristiques soit ont des caractéristiques différentes associées au type ou au volume de gaz introduit dans le contenu C. Ces différentes caractéristiques peuvent inclure, mais de façon non limitative, la dimension et le nombre de trous 35. Selon une variante d'exécution, les moyens 13 d'aération sont tels qu'à un unique élément tubulaire 14a d'amenée est associé un unique élément étendu 15a de distribution. Selon d'autres variantes d'exécution, à un unique élément tubulaire 14a d'amenée sont associés plusieurs éléments étendus 15a de distribution ou, inversement qu'à plusieurs éléments tubulaires 14a d'amenée est associé un unique élément étendu 15a de distribution.

Le au moins un élément étendu 15a de distribution de gaz d'aération peut faire l'objet de différentes variantes d'exécution.

Dans une variante d'exécution, le au moins un élément étendu 15a de distribution de gaz d'aération a, en élévation, une forme générale annulaire ou pseudo annulaire, laquelle a, en section droite transversale, une forme générale qui peut-être circulaire, ou pseudo-circulaire, ou elliptique ou pseudo-elliptique. Un tel au moins un élément annulaire 15a est en communication fluidique avec l'élément tubulaire d'amenée 14a. Selon une variante, à l'élément annulaire 15a est associé au moins un élément transversal, notamment radial, ce qui permet une distribution de gaz plus répartie.

Selon les cas, l'élément annulaire 15a est en forme d'anneau complet fermé sur lui-même, en communication fluidique circulaire continue ou non, ou est en forme anneau incomplet ouvert par rapport à lui-même. Dans une réalisation, l'ouverture d'angle d'un tel anneau ouvert est comprise entre environ 180° et 270°.

Dans une variante d'exécution, un tel élément annulaire 15a est sensiblement centré sur le port de vidange 6.

Dans une variante d'exécution, les moyens 13 d'aération comportent un unique ensemble de moyens 14 d'amenée de gaz d'aération et de moyens 15 de distribution de gaz d'aération. Dans une autre variante d'exécution, les moyens 13 d'aération comportent plusieurs ensembles distincts de moyens 14 d'amenée d'un ou de plusieurs gaz d'aération et de moyens 15 de distribution du ou des gaz d'aération.

Un tel ensemble 14+15 de moyens 13 d'aération peut faire l'objet de différentes variantes d'exécution, en ce que ledit ensemble comporte soit un seul élément tubulaire 14a d'amenée de gaz d'aération communiquant avec un seul élément annulaire 15a de distribution de gaz d'aération, soit un seul élément tubulaire 14a communiquant avec plusieurs éléments annulaire 15a, soit plusieurs éléments tubulaires 14a communiquant avec un seul élément annulaire 15a, soit encore plusieurs éléments tubulaires 14a communiquant avec plusieurs éléments étendus annulaire 15a.

D'autre part, dans le cas où un tel ensemble 14+15 comporte plusieurs éléments annulaire 15a de distribution de gaz d'aération distincts, il est possible, dans une variante d'exécution, que ces - ou certains de ces - éléments annulaire 15a soient situés en plusieurs localisations radiales dans l'espace intérieur 4 et vers la partie inférieure 3a de la paroi 3 du conteneur 2. Dans ce cas, de telles localisations radiales peuvent, dans une variante d'exécution, être substantiellement espacées les unes des autres, radialement par rapport à l'axe XX entre le port de vidange 6 jusqu'au voisinage de la partie latérale 3b de la paroi 3 du conteneur 2.

Avec une telle disposition, l'aération est particulièrement bien répartie, au niveau de sa distribution.

Comme il a été dit précédemment, un élément annulaire 15a de distribution de gaz d'aération est substantiellement espacé radialement du port de vidange 6. Selon une réalisation, cet espacement ou distance est de l'ordre d'au moins le cinquième du diamètre de la partie inférieure de la paroi 3 du conteneur 2.

Aux moyens 13 d'aération qui viennent d'être décrits peuvent être associés fonctionnellement au moins un port d'évacuation de gaz d'aération 36 coopérant avec au moins un orifice d'évacuation ménagé dans la partie supérieure 3c de la paroi 3 du conteneur 2. Un tel port d'évacuation de gaz d'aération 36 peut être pourvu d'une valve anti-retour, empêchant l'introduction dans le conteneur 2 de fluides ou de contaminants non souhaités ou indésirables. Un tel port d'évacuation de gaz d'aération 36 permet d'évacuer du conteneur 2, vers l'extérieur, le gaz qui n'a pas été mélangé dans le contenu du conteneur 2. Un tel port d'évacuation de gaz d'aération 36 peut être en communication fluidique avec l'entrée de gaz d'aération, en vue du recyclage.

Le récipient-mélangeur 1 peut, dans certaines réalisations, comporter également un ou plusieurs ports 37 de montage par exemple d'un moyen fonctionnel, apte à assurer le maintien d'un organe fonctionnel 38 tel que typiquement la collecte ou la mesure de données, la prise d'échantillon aux fins d'analyse.

## Revendications

1. Récipient-mélangeur (1) destiné à recevoir un contenu (C) biopharmaceutique en vue de son mélange, comprenant:
▪ un conteneur (2) flexible, comportant :
∘ une paroi (3) ayant une partie inférieure (3a), une partie latérale (3b) et une partie supérieure (3c), délimitant un espace intérieur (4) apte à recevoir une certaine quantité du contenu (C),
∘ un ou plusieurs ports (5) d'introduction dans le conteneur (2) du contenu (C) ou de composants (C1, C2...) du contenu (C), coopérant avec un ou plusieurs orifices d'introduction ménagés dans le conteneur (2),
∘ au moins un port de vidange (6) du contenu (C) coopérant avec au moins un orifice de vidange,
▪ des moyens de mélange (7) du contenu (C), comportant :
∘ au moins un arbre (8), apte à être entraîné à rotation par des moyens moteur (9) et à entraîner à rotation au moins un organe de mélange (10),
∘ au moins un palier (11) avec lequel coopère une partie d'extrémité de l'arbre (8b),
∘ au moins un organe de mélange (10), apte à agiter le contenu (C), situé dans l'espace intérieur (4),
**caractérisé par le fait qu'**il comporte au moins un port combiné introduction/palier supérieur (5+11a) ayant une flasque (16a) rigide :
∘ pourvue d'un passage d'introduction (30) en communication fluidique d'un côté avec l'espace intérieur (4) et de l'autre avec l'extérieur du conteneur (2),
∘ fixée de façon rigide et étanche à la partie supérieure (3c) de la paroi (3) du conteneur (2) autour d'un orifice d'introduction, le passage d'introduction (30) et l'orifice d'introduction étant en communication fluidique,
∘ supportant du côté intérieur un palier supérieur (11a) situé dans l'espace intérieur (4), adjacent au passage d'introduction (30) sans empêcher la communication fluidique entre le passage d'introduction (30) et l'ouverture d'introduction.

2. Récipient-mélangeur (1) selon la revendication 1, **caractérisé par le fait qu'**il comporte en outre des moyens d'aération (13) aptes à délivrer au contenu (C) une certaine quantité de gaz d'aération, comportant des moyens d'amenée (14) de gaz d'aération ayant au moins un élément tubulaire (14a) s'étendant avec communication fluidique depuis l'extérieur du conteneur (2) jusqu'aux moyens de distribution (15), et des moyens de distribution (15) de gaz d'aération comprenant au moins un élément étendu (15a) de distribution dont la paroi laisse passer des bulles du gaz d'aération provenant des moyens d'amenée (14), situé dans l'espace intérieur (4) vers la partie inférieure (3a) de la paroi (3) du conteneur (2).

3. Récipient-mélangeur (1) selon la revendication 2, dans lequel le au moins un élément tubulaire (14a) d'amenée de gaz d'aération s'étend dans l'espace intérieur (4), en étant substantiellement maintenu attenant ou adjacent à la face intérieure de la paroi (3) du conteneur (2) et traversant celle-ci dans la partie supérieure (3c) par une liaison étanche (33).

4. Récipient-mélangeur (1) selon la revendication 2 ou 3, dans lequel le au moins un élément étendu (15a) de distribution de gaz d'aération est espacé radialement du port de vidange (6).

5. Récipient-mélangeur selon l'une quelconque des revendications 1 à 4, **caractérisé par le fait que** le au moins un arbre (8) est maintenu par le seul palier supérieur (11a) et s'étend sur une partie seulement de la distance entre la partie supérieure (3c) et la partie inférieure (3a) de la paroi (3) du conteneur (2), le moyen moteur (9) d'entraînement à rotation de l'arbre (8) étant situé vers la partie supérieure (3c) de la paroi (3) du conteneur (2).

6. Récipient-mélangeur (1) selon l'une quelconque des revendications 1 à 5, **caractérisé par le.fait que** le moins un organe de mélange (10) est substantiellement espacé de la partie inférieure (3a) et de la partie supérieure (3c) de la paroi (3) du conteneur (2).

7. Récipient-mélangeur (1) selon l'une quelconque des revendications 1 à 6, **caractérisé par le fait que** le au moins un arbre (8) des moyens de mélange (7).est soit situé tout entier dans l'espace intérieur (4), le moyen moteur (9) d'entraînement à rotation de l'arbre (8) étant à fonctionnement magnétique, un disque rotatif menant à pôles magnétiques, situé à l'extérieur du conteneur (2), coopérant fonctionnellement avec un disque rotatif mené à pôles magnétiques, fixé sur le au moins un arbre (8) à proximité magnétique du disque rotatif menant, soit situé pour partie dans l'espace intérieur (4) et pour partie à l'extérieur du conteneur (2), le moyen moteur (9) d'entraînement à rotation de l'arbre (8) étant à fonctionnement mécanique, un arbre (8) rotatif menant, situé à l'extérieur du conteneur (2), coopérant fonctionnellement avec la partie extérieure du au moins un arbre (8).

8. Récipient-mélangeur (1) selon l'une quelconque des revendications 1 à 7, **caractérisé par le fait que** les moyens de mélange (7) comportent soit un unique arbre (8) descendant, soit plusieurs arbres (8) descendant d'axes substantiellement parallèles, aptes à entraîner à rotation chacun au moins un organe de mélange (10).

9. Récipient-mélangeur (1) selon l'une quelconque des revendications 1 à 8, **caractérisé par le fait qu'**un arbre (8) des moyens de mélange (7) supporte et entraîne soit un unique organe de mélange (10) situé en une unique localisation axiale sur l'arbre (8), soit plusieurs organes de mélange (10) situés en une pluralité de localisations axiales sur l'arbre (8).

10. Récipient-mélangeur (1) selon l'une quelconque des revendications 1 à 9, **caractérisé par le fait qu'**un organe de mélange (10) est substantiellement espacé de la partie supérieure (3c) de la paroi (3) du conteneur (2), d'une distance de l'ordre d'au moins le tiers de l'écartement entre la partie inférieure (3a) et la partie supérieure (3c) de la paroi (3) du conteneur (2).

11. Récipient-mélangeur (1) selon l'une quelconque des revendications 2 à 4, **caractérisé par le fait que** le au moins un élément tubulaire (14a) d'amenée de gaz d'aération est au moins pour partie structurellement distinct de la paroi (3) du conteneur (2) et maintenue à elle par collage, soudage ou au moyen de pièces de maintien rapportées.

12. Récipient-mélangeur (1) selon l'une quelconque des revendications 2 à 4, **caractérisé par le fait que** le au moins un élément tubulaire (14a) d'amenée de gaz d'aération est au moins pour partie structurellement partie intégrante de la paroi (3) du conteneur (2).

13. Récipient-mélangeur (1) selon l'une quelconque des revendications 2 à 4, **caractérisé par le fait que** le au moins un élément étendu (15a) de distribution de gaz d'aération est maintenu attenant ou adjacent à la face intérieure de la partie inférieure (3a) de la paroi (3) du conteneur (2).

14. Récipient-mélangeur (1) selon l'une quelconque des revendications 2 à 4, **caractérisé par le fait que** le au moins un élément étendu (15a) de distribution de gaz d'aération est, au moins pour partie, soit structurellement distinct de la paroi (3) du conteneur (2) et maintenu à elle par collage, soudage ou au moyen de pièces de maintien rapportées, soit structurellement partie intégrante de la paroi (3) du conteneur (2).

15. Récipient-mélangeur (1) selon l'une quelconque des revendications 2 à 4, **caractérisé par le fait que** le au moins un élément étendu (15a) de distribution de gaz d'aération ne traverse pas la paroi (3) du conteneur (2).

16. Récipient-mélangeur (1) selon l'une quelconque des revendications 2 à 4, **caractérisé par le fait que** le au moins un élément étendu (15a) de distribution de gaz d'aération comporte une paroi pourvue d'une pluralité de trous (35) répartis aptes au passage des bulles du gaz d'aération provenant des moyens d'amenée (14) et, en particulier avec la pluralité de trous (35) aptes au passage des bulles du gaz d'aération provenant des moyens d'amenée (14) orientée avec différents axes d'inclinaison sur la verticale, les trous de la pluralité de trous (35) étant soit de même taille soit de tailles différentes.

17. Récipient-mélangeur (1) selon l'une quelconque des revendications 2 à 4, **caractérisé par le fait que** le au moins un élément étendu (15a) de distribution de gaz d'aération a, en section droite transversale, une forme circulaire, ou pseudo-circulaire, ou elliptique ou pseudo-elliptique.

18. Récipient-mélangeur (1) selon l'une quelconque des revendications 2 à 4, **caractérisé par le fait que** le au moins un élément étendu (15a) de distribution de gaz d'aération comprend soit au moins un anneau complet fermé sur lui-même, en communication circulaire continue ou non, soit au moins un au moins un anneau incomplet ouvert par rapport à lui-même, en particulier avec une ouverture d'angle comprise entre environ 180 ° et 270 °.

19. Récipient-mélangeur (1) selon l'une quelconque des revendications 2 à 4, **caractérisé par le fait que** le au moins un élément étendu (15a) de distribution de gaz d'aération comprend au moins un anneau et au moins un élément transversal en communication fluidique.

20. Récipient-mélangeur (1) selon l'une quelconque des revendications 18 et 19, **caractérisé par le fait que** le au moins un anneau du au moins un élément étendu (15a) de distribution de gaz d'aération est sensiblement centré sur le port de vidange (6).

21. Récipient-mélangeur (1) selon l'une quelconque des revendications 2 à 4, **caractérisé par le fait que** les moyens d'aération (13) comportent soit un unique ensemble de moyens d'amenée (14) de gaz d'aération et de moyens de distribution (15) de gaz d'aération, soit plusieurs ensembles distincts de moyens d'amenée (14) d'un ou de plusieurs gaz d'aération et de moyens de distribution (15) du ou des gaz d'aération.

22. Récipient-mélangeur (1) selon la revendication 21, **caractérisé par le fait qu'**un ensemble de moyens d'aération (13) comporte un seul élément tubulaire (14a) d'amenée de gaz d'aération communiquant avec un seul élément étendu (15a) de distribution de gaz d'aération, ou un seul élément tubulaire (14a) d'amenée de gaz d'aération communiquant avec plusieurs éléments étendus (15a) de distribution de gaz d'aération, ou plusieurs éléments tubulaires d'amenée (14a) dé gaz d'aération communiquant avec un seul élément étendu (15a) de distribution de gaz d'aération, ou plusieurs éléments tubulaires d'amenée (14a) de gaz d'aération communiquant avec plusieurs éléments étendus (15a) de distribution de gaz d'aération.

23. Récipient-mélangeur (1) selon l'une quelconque des revendications 2 à 4, dans le cas où il comporte plusieurs éléments étendus (15a) de distribution de gaz d'aération distincts, **caractérisé par le fait que** au moins certains des plusieurs éléments étendus (15a) de distribution de gaz d'aération sont situés en une pluralité de localisations radiales dans l'espace intérieur (4) vers la partie inférieure (3a) du conteneur (2).

24. Récipient-mélangeur (1) selon la revendication 23, **caractérisé par le fait que** les plusieurs éléments étendus (15a) de distribution de gaz d'aération distincts sont substantiellement espacés radialement du port de vidange (6) jusqu'au voisinage de la partie latérale (3b) de la paroi (3) du conteneur (2).

25. Récipient-mélangeur (1) selon l'une quelconque des revendications 2 à 4, **caractérisé par le fait qu'**un élément étendu (15a) de distribution de gaz d'aération est substantiellement espacé radialement du port de vidange (6) d'une distance de l'ordre d'au moins le cinquième du diamètre de la partie inférieure (3a) de la paroi (3) du conteneur (2).

26. Récipient-mélangeur (1) selon l'une quelconque des revendications 1 à 25, **caractérisé par le fait que** ne saille sous la partie inférieure (3a) de la paroi (3) du conteneur (2) que la vidange.

27. Récipient-mélangeur (1) selon l'une quelconque des revendications 2 à 4, **caractérisé par le fait qu'**il comporte également un ou plusieurs ports d'évacuation de gaz coopérant avec au moins un orifice d'évacuation ménagé dans la partie supérieure (3c) de la paroi (3) du conteneur (2), pourvu d'une valve anti-retour, empêchant l'introduction dans le conteneur (2) de fluides ou de contaminants non souhaités ou indésirables.

28. Récipient-mélangeur (1) selon l'une quelconque des revendications 1 à 27, **caractérisé par le fait qu'**il comporte également un ou plusieurs ports d'introduction, de vidange, de montage.

29. Récipient-mélangeur (1) selon l'une quelconque des revendications 1 à 28, **caractérisé par le fait que** le conteneur (2) est de grande capacité, pouvant aller jusqu'à 5.000 litres.

30. Récipient-mélangeur (1) selon l'une quelconque des revendications 1 à 29, **caractérisé par le fait qu'**il comporte également un dispositif rigide extérieur de contention (18) du conteneur (2) empli de son contenu (C), comprenant une paroi de fond (19), une paroi périphérique (20) et une ouverture supérieure (21), délimitant un logement principal dans lequel est placé de façon amovible le conteneur (2) flexible dont la partie inférieure (3a) repose sur la paroi de fond (19) et dont la partie latérale (3b) vient s'appliquer, lorsque le conteneur (2) est empli de son contenu (C), contre la paroi périphérique (20).

31. Récipient-mélangeur (1) selon la revendication 30, **caractérisé par le fait que** le dispositif rigide extérieur de contention (18) comporte également un logement secondaire en dessous de la paroi de fond (19) de logement et de protection de la vidange et, le cas échéant, du moyen moteur (9) d'entraînement des moyens de mélange (7) lorsqu'il est prévu en partie inférieure (3a) et/ou des moyens de chauffage et le conteneur (2) flexible est en un matériau présentant une certaine conductivité thermique, de manière que la mise en oeuvre des moyens de chauffage permette le chauffage du contenu (C) ; et, le cas échéant, des moyens de contrôle de la température du conteneur (2) et des moyens de commande des moyens de chauffage.

32. Récipient-mélangeur (1) selon l'une quelconque des revendications 30 et 31, **caractérisé par le fait que** le conteneur (2) peut se trouver dans trois états extrêmes: un état désassemblé du dispositif rigide extérieur de contention (18) dans lequel le conteneur (2) peut être disposé de façon aplatie sur lui-même, un état assemblé au dispositif rigide extérieur de contention (18) dans lequel le conteneur (2), vide de contenu (Ç), est disposé dans le logement principal du dispositif de contention en reposant sur la paroi de fond (19), et un état assemblé au dispositif rigide extérieur de contention (18) dans lequel le conteneur (2), empli de son contenu (C), est disposé dans le logement principal du dispositif de contention en reposant sur la paroi de fond (19) et en étant appliqué contre la paroi périphérique (20).

33. Récipient-mélangeur (1) selon l'une quelconque des revendications 1 à 32, **caractérisé par le fait que** l'on y réalise une bioréaction, le récipient-mélangeur (1) étant un bioréacteur.

## Patentansprüche

1. Mischbehälter (1), der dazu bestimmt ist, einen biopharmazeutischen Inhalt (C) aufzunehmen, um ihn zu mischen, umfassend:
▪ einen flexiblen Behälter (2), umfassend:
∘ eine Wand (3), die einen unteren Teil (3a), einen seitlichen Teil (3b) und einen oberen Teil (3c) hat und die einen inneren Raum (4) begrenzt, der geeignet ist, eine bestimmte Menge des Inhalts (C) aufzunehmen,
∘ einen oder mehrere Anschlüsse (5) zum Einführen des Inhalts (C) oder von Bestandteilen (C1, C2...) des Inhalts (C) in den Behälter (2), der/die mit einer oder mehreren Öffnungen zum Einführen zusammenwirkt/zusammenwirken, die im Behälter (2) angeordnet sind,
∘ mindestens einen Anschluss zum Entleeren (6) des Inhalts (C), der mit mindestens einer Öffnung zum Entleeren zusammenwirkt,
▪ Mittel zum Mischen (7) des Inhalts (C), umfassend:
∘ mindestens eine Welle (8), die geeignet ist, von Motormitteln (9) drehend angetrieben zu werden und mindestens ein Mischorgan (10) drehend anzutreiben,
∘ mindestens ein Lager (11), mit dem ein Teil eines Wellenendes (8b) zusammenwirkt,
∘ mindestens ein Mischorgan (10), das geeignet ist, den Inhalt (C), der sich in dem inneren Raum (4) befindet, zu rühren,
**dadurch gekennzeichnet, dass** er mindestens eine Kombination aus dem Anschluss zum Einführen und dem oberen Lager (5+11 a) mit einem steifen Flansch (16a) umfasst:
∘ der mit einem Durchgang zum Einführen (30) versehen ist, der auf der einen Seite mit dem inneren Raum (4) und auf der anderen mit der Außenseite des Behälters (2) über eine Fluidverbindung verbunden ist,
∘ der steif und abgedichtet am oberen Teil (3c) der Wand (3) des Behälters (2) um eine Öffnung zum Einführen befestigt ist, wobei der Durchgang zum Einführen (30) und die Öffnung zum Einführen eine Fluidverbindung aufweisen,
∘ der auf der inneren Seite ein oberes Lager (11a) trägt, das im inneren Raum (4) liegt, das an den Durchgang zum Einführen (30) angrenzt, ohne die Fluidverbindung zwischen dem Durchgang zum Einführen (30) und der Öffnung zum Einführen zu unterbinden.

2. Mischbehälter (1) nach Anspruch 1, **dadurch gekennzeichnet, dass** er ferner Mittel zum Belüften (13), die geeignet sind, dem Inhalt (C) eine bestimmte Menge an Belüftungsgas bereitzustellen, umfassend Mittel zum Zuführen (14) von Belüftungsgas, die mindestens ein röhrenförmiges Element (14a) aufweisen, das sich in Fluidverbindung von der Außenseite des Behälters (2) bis zu den Mitteln zum Verteilen (15) erstreckt, und Mittel zum Verteilen (15) von Belüftungsgas umfasst, die mindestens ein verlängertes Element (15a) zum Verteilen umfassen, dessen Wand die Blasen des Belüftungsgases durchlässt, das aus den Mitteln zum Zuführen (14) stammt, das im inneren Raum (4) in Richtung des unteren Teils (3a) der Wand (3) des Behälters (2) liegt.

3. Mischbehälter (1) nach Anspruch 2, wobei sich das mindestens eine röhrenförmige Element (14a) zum Zuführen von Belüftungsgas in den inneren Raum (4) erstreckt und es dabei im Wesentlichen anliegend oder angrenzend an die innere Seite der Wand (3) des Behälters (2) gehalten wird und es diese in dem oberen Teil (3c) mit einer dichten Verbindung (33) durchdringt.

4. Mischbehälter (1) nach Anspruch 2 oder 3, wobei das mindestens eine verlängerte Element (15a) zum Verteilen von Belüftungsgas vom Anschluss zum Entleeren (6) radial beabstandet ist.

5. Mischbehälter nach einem der Ansprüche 1 bis 4, **dadurch gekennzeichnet, dass** die mindestens eine Welle (8) nur von dem oberen Lager (11a) gehalten wird und sich nur über einen Teil der Entfernung zwischen dem oberen Teil (3c) und dem unteren Teil (3a) der Wand (3) des Behälters (2) erstreckt, wobei das Motormittel (9) zum drehenden Antreiben der Welle (8) in Richtung des oberen Teils (3c) der Wand (3) des Behälters (2) liegt.

6. Mischbehälter (1) nach einem der Ansprüche 1 bis 5, **dadurch gekennzeichnet, dass** das mindestens eine Mischorgan (10) von dem unteren Teil (3a) und dem oberen Teil (3c) der Wand (3) des Behälters (2) wesentlich beabstandet ist.

7. Mischbehälter (1) nach einem der Ansprüche 1 bis 6, **dadurch gekennzeichnet, dass** die mindestens eine Welle (8) der Mittel zum Mischen (7) entweder vollständig im inneren Raum (4) liegt, wobei das Motormittel (9) zum drehenden Antreiben der Welle (8) im Magnetbetrieb arbeitet, wobei eine Drehantriebsscheibe mit magnetischen Polen, die außerhalb des Behälters (2) liegt, die funktionell mit einer Drehantriebsscheibe, die von magnetischen Polen angetrieben wird, zusammenwirkt, auf der mindestens einen Welle (8) in magnetischer Nähe zu der Drehantriebsscheibe befestigt ist, oder teilweise im inneren Raum (4) und teilweise außerhalb des Behälters (2) liegt, wobei das Motormittel (9) zum drehenden Antreiben der Welle (8) mechanisch arbeitet, wobei eine Drehantriebswelle (8), die außerhalb des Behälters (2) liegt, mit dem außen liegenden Teil der mindestens einen Welle (8) funktionell zusammenwirkt.

8. Mischbehälter (1) nach einem der Ansprüche 1 bis 7, **dadurch gekennzeichnet, dass** die Mittel zum Mischen (7) entweder eine einzige abwärts weisende Welle (8) oder mehrere abwärts weisende Wellen (8) mit im Wesentlichen parallelen Achsen umfassen, die jeweils geeignet sind, mindestens ein Mischorgan (10) drehend anzutreiben.

9. Mischbehälter (1) nach einem der Ansprüche 1 bis 8, **dadurch gekennzeichnet, dass** eine Welle (8) der Mittel zum Mischen (7) entweder ein einziges Mischorgan (10), das sich an einer einzigen axialen Stelle auf der Welle (8) befindet, oder mehrere Mischorgane (10), die sich an einer Vielzahl von axialen Stellen auf der Welle (8) befinden, trägt und antreibt.

10. Mischbehälter (1) nach einem der Ansprüche 1 bis 9, **dadurch gekennzeichnet, dass** ein Mischorgan (10) vom oberen Teil (3c) der Wand (3) des Behälters (2), in einer Entfernung in der Größenordnung von mindestens einem Drittel des Abstands zwischen dem unteren Teil (3a) und dem oberen Teil (3c) der Wand (3) des Behälters (2), wesentlich beabstandet ist.

11. Mischbehälter (1) nach einem der Ansprüche 2 bis 4, **dadurch gekennzeichnet, dass** das mindestens eine röhrenförmige Element (14a) zum Zuführen von Belüftungsgas mindestens teilweise von der Wand (3) des Behälters (2) strukturell verschieden ist und an ihr durch Verkleben, Verschweißen oder mittels angesetzter Halteteile gehalten wird.

12. Mischbehälter (1) nach einem der Ansprüche 2 bis 4, **dadurch gekennzeichnet, dass** das mindestens eine röhrenförmige Element (14a) zum Zuführen von Belüftungsgas mindestens teilweise ein strukturell fester Bestandteil der Wand (3) des Behälters (2) ist.

13. Mischbehälter (1) nach einem der Ansprüche 2 bis 4, **dadurch gekennzeichnet, dass** das mindestens eine verlängerte Element (15a) zum Verteilen von Belüftungsgas angrenzend oder anliegend an der inneren Seite des unteren Teils (3a) der Wand (3) des Behälters (2) gehalten wird.

14. Mischbehälter (1) nach einem der Ansprüche 2 bis 4, **dadurch gekennzeichnet, dass** das mindestens eine verlängerte Element (15a) zum Verteilen von Belüftungsgas mindestens teilweise entweder strukturell von der Wand (3) des Behälters (2) verschieden ist und an ihr durch Verkleben, Verschweißen oder mittels angesetzter Halteteile gehalten wird, oder fester Bestandteil der Wand (3) des Behälters (2) ist.

15. Mischbehälter (1) nach einem der Ansprüche 2 bis 4, **dadurch gekennzeichnet, dass** das mindestens eine verlängerte Element (15a) zum Verteilen von Belüftungsgas die Wand (3) des Behälters (2) nicht durchdringt.

16. Mischbehälter (1) nach einem der Ansprüche 2 bis 4, **dadurch gekennzeichnet, dass** das mindestens eine verlängerte Element (15a) zum Verteilen von Belüftungsgas eine Wand umfasst, die mit einer Vielzahl von verteilten Löchern (35) versehen ist, die für den Durchgang der Blasen des Belüftungsgases, das aus den Mitteln zum Zuführen (14) stammt, geeignet sind, und insbesondere mit einer Vielzahl von Löchern (35), die für den Durchgang der Blasen des Belüftungsgases, das aus den Mitteln zum Zuführen (14) stammt, geeignet sind, die mit verschiedenen Neigungsachsen zur Vertikalen ausgerichtet sind, wobei die Löcher der Vielzahl von Löchern (35) entweder die gleiche Größe oder verschiedene Größen haben.

17. Mischbehälter (1) nach einem der Ansprüche 2 bis 4, **dadurch gekennzeichnet, dass** das mindestens eine verlängerte Element (15a) zum Verteilen von Belüftungsgas im Querschnitt eine kreisförmige oder pseudo-kreisförmige oder elliptische oder pseudoelliptische Form hat.

18. Mischbehälter (1) nach einem der Ansprüche 2 bis 4, **dadurch gekennzeichnet, dass** das mindestens eine verlängerte Element (15a) zum Verteilen von Belüftungsgas entweder mindestens einen vollständigen, in sich geschlossenen Ring in kreisförmiger, durchgehender Verbindung oder nicht oder mindestens einen unvollständigen, in sich offenen Ring, insbesondere mit einer Winkelöffnung von einschließlich etwa 180° bis 270°, umfasst.

19. Mischbehälter (1) nach einem der Ansprüche 2 bis 4, **dadurch gekennzeichnet, dass** das mindestens eine verlängerte Element (15a) zum Verteilen von Belüftungsgas mindestens einen Ring und mindestens ein Querelement in Fluidverbindung umfasst.

20. Mischbehälter (1) nach einem der Ansprüche 18 und 19, **dadurch gekennzeichnet, dass** der mindestens eine Ring des mindestens einen verlängerten Elements (15a) zum Verteilen von Belüftungsgas im Wesentlichen auf dem Anschluss zum Entleeren (6) zentriert ist.

21. Mischbehälter (1) nach einem der Ansprüche 2 bis 4, **dadurch gekennzeichnet, dass** die Mittel zum Belüften (13) entweder eine einzige Gruppe von Mitteln zum Zuführen (14) von Belüftungsgas und von Mitteln zum Verteilen (15) von Belüftungsgas oder mehrere unterschiedliche Gruppen von Mitteln zum Zuführen (14) von einem oder mehreren Belüftungsgas(en) und Mitteln zum Verteilen (15) des oder der Belüftungsgase(s) umfassen.

22. Mischbehälter (1) nach Anspruch 21, **dadurch gekennzeichnet, dass** eine Gruppe von Mitteln zum Belüften (13) ein einziges röhrenförmiges Element (14a) zum Zuführen von Belüftungsgas, das mit einem einzigen verlängerten Element (15a) zum Verteilen von Belüftungsgas verbunden ist, oder ein einziges röhrenförmiges Element (14a) zum Zuführen von Belüftungsgas, das mit mehreren verlängerten Elementen (15a) zum Verteilen von Belüftungsgas verbunden ist, oder mehrere röhrenförmige Elemente zum Zuführen (14a) von Belüftungsgas, die mit einem einzigen verlängerten Element (15a) zum Verteilen von Belüftungsgas verbunden sind oder mehrere röhrenförmige Elemente zum Zuführen (14a) von Belüftungsgas, die mit mehreren verlängerten Elementen (15a) zum Verteilen von Belüftungsgas verbunden sind, umfasst.

23. Mischbehälter (1) nach einem der Ansprüche 2 bis 4, wenn er mehrere unterschiedliche verlängerte Elemente (15a) zum Verteilen von Belüftungsgas umfasst, **dadurch gekennzeichnet, dass** mindestens einige der mehreren verlängerten Elemente (15a) zum Verteilen von Belüftungsgas an einer Vielzahl von radial verteilten Stellen in dem inneren Raum (4) in Richtung zum unteren Teil (3a) des Behälters (2) liegen.

24. Mischbehälter (1) nach Anspruch 23, **dadurch gekennzeichnet, dass** die mehreren unterschiedlichen verlängerten Elemente (15a) zum Verteilen von Belüftungsgas vom Anschluss zum Entleeren (6) bis in die Nähe des seitlichen Teils (3b) der Wand (3) des Behälters (2) wesentlich radial beabstandet sind.

25. Mischbehälter (1) nach einem der Ansprüche 2 bis 4, **dadurch gekennzeichnet, dass** ein verlängertes Element (15a) zum Verteilen von Belüftungsgas vom Anschluss zum Entleeren (6) in einer Entfernung in der Größenordnung von mindestens einem Fünftel des Durchmessers des unteren Teiles (3a) der Wand (3) des Behälters (2) wesentlich radial beabstandet ist.

26. Mischbehälter (1) nach einem der Ansprüche 1 bis 25, **dadurch gekennzeichnet, dass** nur die Entleerung unter dem unteren Teil (3a) der Wand (3) des Behälters (2) vorsteht.

27. Mischbehälter (1) nach einem der Ansprüche 2 bis 4, **dadurch gekennzeichnet, dass** er auch einen Anschluss oder mehrere Anschlüsse zum Ablassen von Gas umfasst, der/die mit mindestens einer Öffnung zum Ablassen zusammenwirkt/zusammenwirken, die im oberen Teil (3c) der Wand (3) des Behälters (2) angeordnet ist und mit einem Rückschlagventil versehen ist, das das Einführen von nicht erwünschten oder unerwünschten Fluiden oder Verunreinigungen in den Behälter (2) verhindert.

28. Mischbehälter (1) nach einem der Ansprüche 1 bis 27, **dadurch gekennzeichnet, dass** er auch einen oder mehrere Anschlüsse zum Einführen, zum Entleeren, zum Zusammenbauen umfasst.

29. Mischbehälter (1) nach einem der Ansprüche 1 bis 28, **dadurch gekennzeichnet, dass** der Behälter (2) ein großes Fassungsvermögen aufweist, das bis zu 5.000 Liter beinhalten kann.

30. Mischbehälter (1) nach einem der Ansprüche 1 bis 29, **dadurch gekennzeichnet, dass** er auch eine äußere steife Vorrichtung zum Halten (18) des mit seinem Inhalt (C) gefüllten Behälters (2) umfasst, mit einem Boden (19), einer Umfangswand (20) und einer oberen Öffnung (21), die eine Hauptaufnahme begrenzt, in der der flexible Behälter (2) entnehmbar angeordnet ist, dessen unterer Teil (3a) auf dem Boden (19) ruht und dessen seitlicher Teil (3b) gegen die Umfangswand (20) zur Anlage kommt, wenn der Behälter (2) mit seinem Inhalt (C) gefüllt ist.

31. Mischbehälter (1) nach Anspruch 30, **dadurch gekennzeichnet, dass** die äußere steife Vorrichtung zum Halten (18) auch eine zweite Aufnahme unterhalb des Bodens (19) zum Aufnehmen und zum Schützen der Entleerung, und gegebenenfalls des Motormittels (9) zum Antreiben der Mittel zum Mischen (7), wenn es im unteren Teil (3a) vorgesehen ist, und/oder der Mittel zum Erwärmen umfasst, und der flexible Behälter (2) aus einem Material ist, das eine bestimmte thermische Leitfähigkeit aufweist, so dass das Einsetzen der Mittel zum Erwärmen das Erwärmen des Inhalts (C) ermöglicht; und gegebenenfalls der Mittel zum Kontrollieren der Temperatur des Behälters (2) und der Mittel zum Steuern der Mittel zum Erwärmen.

32. Mischbehälter (1) nach einem der Ansprüche 30 und 31, **dadurch gekennzeichnet, dass** sich der Behälter (2) in drei Endzuständen befinden kann: einem demontierten Zustand der äußeren steifen Vorrichtung zum Halten (18), in dem der Behälter (2) flach auf sich selbst angeordnet sein kann, einem mit der äußeren steifen Vorrichtung zum Halten (18) zusammengebauten Zustand, in dem der Behälter (2) ohne Inhalt (C) in der Hauptaufnahme der Vorrichtung zum Halten angeordnet ist, indem er auf dem Boden (19) ruht, und einem mit der äußeren steifen Vorrichtung zum Halten (18) zusammengebauten Zustand, in dem der mit seinem Inhalt (C) gefüllte Behälter (2) in der Hauptaufnahme der Vorrichtung zum Halten angeordnet ist und auf dem Boden (19) ruht und sich in Anlage an die Umfangswand (20) befindet.

33. Mischbehälter (1) nach einem der Ansprüche 1 bis 32, **dadurch gekennzeichnet, dass** darin eine Bioreaktion durchgeführt wird, wobei der Mischbehälter (1) ein Bioreaktor ist.

## Claims

1. Mixing vessel (1) that is designed to accommodate biopharmaceutical contents (C) (C) for the purpose of the mixing thereof, comprising:
▪ A flexible container (2), comprising:
∘ A wall (3) that has a lower part (3a), a lateral part (3b), and an upper part (3c), delimiting an interior space (4) that can accommodate a certain amount of the contents (C),
∘ One or more ports (5) for introducing contents (C) or components (C1, C2...) of contents (C) into the container (2), working with one or more introduction openings provided in the container (2),
∘ At least one port (6) for draining the contents (C), working with at least one drain opening,
▪ Means for mixing (7) the contents (C), comprising:
∘ At least one shaft (8), able to be driven in rotation by motor means (9) and to drive in rotation at least one mixing element (10),
∘ At least one bearing (11) with which one end part of the shaft (8b) works,
∘ At least one mixing element (10), able to stir the contents (C), located in the interior space (4),
**characterized by** the fact that it comprises at least one combined introduction/upper bearing port (5+11 a) having a rigid flange (16a):
∘ Provided with an introduction passage (30) in fluid communication with the interior space (4), on the one hand, and with the exterior of the container (2), on the other hand,
∘ Attached in a rigid and airtight way to the upper part (3c) of the container wall (3) around an introduction opening, with the introduction passage (30) and the introduction opening being in fluid communication,
∘ Supporting - from the interior side - an upper bearing (11 a) that is located in the interior space (4), adjacent to the introduction passage (30) without preventing the fluid communication between the introduction passage (30) and the introduction opening.

2. Mixing vessel (1) according to Claim 1, wherein it also comprises aeration means (13) that can deliver to the contents (C) a certain amount of aeration gas, comprising aeration gas intake means (14) having at least one tubular element (14a) that extends with fluid communication from the exterior of the container (2) to the distribution means (15) and aeration gas distribution means (15) comprising at least one extended distribution element (15a) whose wall can allow aeration gas bubbles originating from the intake means (14) to pass, whereby said element is located in the interior space (4) toward the lower part (3a) of the container wall (3).

3. Mixing vessel (1) according to claim 2, wherein the at least one tubular aeration gas intake element (14a) extends into the interior space (4) by being held substantially adjoining or adjacent to the interior surface of the container wall (3) and running through the latter in the upper part (3c) by an airtight connection (33).

4. Mixing vessel (1) according to claim 2 or 3, wherein the at least one extended aeration gas distribution element (15a) is radially spaced from the drain port (6).

5. Mixing vessel (1) according to any of Claims 1 to 4, wherein the at least one shaft (8) is held by the single upper bearing (11 a) and extends over only a part of the distance between the upper part (3c) and the lower part (3a) of the container wall (3), whereby the motor means (9) for driving the shaft (8) in rotation is located toward the upper part (3c) of the container wall (3).

6. Mixing vessel (1) according to any of Claims 1 to 5, wherein the at least one mixing element (10) is spaced substantially far away from the lower part (3a) and the upper part (3c) of the container wall (3).

7. Mixing vessel (1) according to any of Claims 1 to 6, wherein the at least one shaft (8) of the mixing means (7) is either located in its entirety in the interior space (4), with the motor means (9) for driving the shaft (8) in rotation operating magnetically, a rotary disk being driven with magnetic poles, where said disk is located on the exterior of the container (2), operationally works with a rotary disk driven with magnetic poles, and is attached to the at least one shaft (8) in magnetic proximity to the driving rotary disk, or is partly located in the interior space (4) and partly on the exterior of the container (2), with the motor means (9) driving the shaft (8) in rotation operating mechanically, a driving rotary shaft (8), located on the exterior of the container (2), operationally working with the exterior part of at least one shaft (8).

8. Mixing vessel (1) according to any of Claims 1 to 7, wherein the mixing means (7) comprise either a single descending shaft (8) or several shafts (8) descending from substantially parallel axes, each able to drive in rotation at least one mixing element (10).

9. Mixing vessel (1) according to any of Claims 1 to 8, wherein a shaft (8) of the mixing means (7) supports and drives either a single mixing element (10) that is located in a single axial location on the shaft (8) or several mixing elements (10) that are located at a large number of axial locations on the shaft (8).

10. Mixing vessel (1) according to any of Claims 1 to 9, wherein a mixing element (10) is spaced substantially far away from the upper part (3c) of the container wall (3), at a distance on the order of at least one third of the separation between the lower part (3a) and the upper part (3c) of the container wall (3).

11. Mixing vessel (1) according to any of Claims 2 to 4, wherein the at least one tubular aeration gas intake element (14a) is at least partly structurally separate from the container wall (3) and held to it by gluing, welding, or by means of connected holding pieces.

12. Mixing vessel (1) according to any of Claims 2 to 4, wherein the at least one tubular aeration gas intake element (14a) is at least partly structurally an integral part of the container wall (3).

13. Mixing vessel (1) according to any of Claims 2 to 4, wherein the at least one extended aeration gas distribution element (15a) is held adjoining or adjacent to the interior surface of the lower part (3a) of the container wall (3).

14. Mixing vessel (1) according to any of Claims 2 to 4, wherein the at least one extended aeration gas distribution element (15a) is, at least partly, either structurally separate from the container wall (3) and held to it by gluing, welding, or by means of connected holding pieces, or structurally an integral part of the container wall (3).

15. Mixing vessel (1) according to any of Claims 2 to 4, wherein the at least one extended aeration gas distribution element does not pass through the wall (3) of the container (2).

16. Mixing vessel (1) according to any of Claims 2 to 4, wherein the at least one extended aeration gas distribution element (15a) comprises a wall that is provided with a large number of dispersed holes (35) that can allow aeration gas bubbles originating from the intake means (14) to pass, and, in particular with the large number of holes (35) that can allow aeration gas bubbles originating from the intake means (14) to pass, is oriented with different axes of inclination with respect to the vertical line, with the holes of the large number of holes (35) being either the same size or different sizes.

17. Mixing vessel (1) according to any of Claims 2 to 4, wherein the at least one extended aeration gas distribution element (15a) has, in a transverse straight cross-section, a circular or pseudo-circular, or elliptical or pseudo-elliptical shape.

18. Mixing vessel (1) according to any of Claims 2 to 4, wherein the at least one extended aeration gas distribution element (15a) comprises either at least one complete ring that is closed on itself, in circular communication that may or may not be continuous, or at least one incomplete ring that is opened relative to itself, in particular with an angular opening of between approximately 180° and 270°.

19. Mixing vessel (1) according to any of Claims 2 to 4, wherein the at least one extended aeration gas distribution element (15a) comprises at least one ring and at least one transverse element in fluid communication.

20. Mixing vessel (1) according to any of Claims 18 and 19, wherein the at least one ring of at least one extended aeration gas distribution element (15a) is essentially centered on the drain port (6).

21. Mixing vessel (1) according to any of Claims 2 to 4, wherein the aeration means (13) comprise either a single set of aeration gas intake means (14) and aeration gas distribution means (15) or several separate sets of intake means (14) of one or more aeration gases and means (15) for distribution of aeration gas(es).

22. Mixing vessel (1) according to Claim 21, wherein a set of aeration means (13) comprises a single tubular aeration gas intake element (14a) that communicates with a single extended aeration gas distribution element (15a) or a single tubular aeration gas intake element (14a) that communicates with several extended aeration gas distribution elements (15a), or several tubular aeration gas intake elements (14a) that communicate with a single extended aeration gas distribution element (15a), or several tubular aeration gas intake elements (14a) that communicate with several extended aeration gas distribution elements (15a).

23. Mixing vessel (1) according to any of Claims 2 to 4, in the case where it comprises several separate extended aeration gas distribution elements (15a), wherein at least some of the several extended aeration gas distribution elements (15a) are located in a large number of radial locations in the interior space (4) toward the lower part (3a) of the container (2).

24. Mixing vessel (1) according to Claim 23, wherein the several separate extended aeration gas distribution elements (15a) are radially spaced substantially far away from the drain port (6) to the vicinity of the lateral part (3b) of the container wall (3).

25. Mixing vessel (1) according to any of Claims 2 to 4, wherein an extended aeration gas distribution element (15a) is radially spaced substantially far away from the drain port (6) by a distance on the order of at least one-fifth of the diameter of the lower part (3a) of the container wall (3).

26. Mixing vessel (1) according to any of Claims 1 to 25, wherein only the drain projects under the lower part (3a) of the container wall (3).

27. Mixing vessel (1) according to any of Claims 2 to 4, wherein it also comprises one or more gas evacuation ports that work with at least one evacuation opening provided in the upper part (3c) of the container wall (3), provided with a nonreturn valve, preventing the introduction of unwanted or undesirable fluids or contaminants into the container (2).

28. Mixing vessel (1) according to any of Claims 1 to 27, wherein it also comprises one or more ports for introduction, draining, and assembly.

29. Mixing vessel (1) according to any of Claims 1 to 28, wherein the container (2) has a large capacity and can range up to 5,000 liters.

30. Mixing vessel (1) according to any of Claims 1 to 29, wherein it also comprises an external rigid holding device (18) of the container (2) that is filled with its contents (C), comprising a bottom wall (19), a peripheral wall (20), and an upper opening (21), delimiting a primary housing in which the flexible container (2), whose interior part (3a) rests on the bottom wall (19) and whose lateral part (3b) is applied, when the container (2) is filled with its contents (C), against the peripheral wall (20), is arranged in a removable manner.

31. Mixing vessel (1) according to Claim 30, wherein the external rigid holding device (18) also comprises a secondary housing below the bottom wall (19) for housing and for protection of the drain and, if necessary, the drive motor means (9) of the mixing means (7) when it is provided in the lower part (3a) and/or heating means, and the flexible container (2) is made of a material that has a certain thermal conductivity such that the implementation of the heating means makes it possible to heat the contents (C), and, if necessary, means for monitoring the temperature of the container (2) and means for controlling the heating means.

32. Mixing vessel (1) according to any of Claims 30 and 31, wherein the container (2) can be found in three extreme states: a disassembled state of the external rigid holding device (18) in which the container (2) can be arranged flattened on itself; an assembled state of the external rigid holding device (18) in which the container (2), empty of contents (C), is arranged in the primary housing of the holding device by resting on the bottom wall (19); and an assembled state of the external rigid holding device (18) in which the container (2), filled with its contents (C), is arranged in the primary housing of the holding device by resting on the bottom wall (19) and by being applied against the peripheral wall (20).

33. Mixing vessel (1) according to any of Claims 1 to 32, wherein a bioreaction is produced there, with the mixing vessel (1) being a bioreactor.
